Europäisches Patentamt

European Patent Office

Office européen des brevets

(11) Publication number: **0 438 231 A2**

# EUROPEAN PATENT APPLICATION

(21) Application number: **91300185.5**

(51) Int. Cl.5: **C12N 15/76**

(22) Date of filing: **11.01.91**

(30) Priority: **19.01.90 US 467582**

(43) Date of publication of application:
**24.07.91 Bulletin 91/30**

(84) Designated Contracting States:
**AT BE CH DE DK ES FR GB GR IT LI NL SE**

(71) Applicant: **ELI LILLY AND COMPANY**
**Lilly Corporate Center**
**Indianapolis Indiana 46285(US)**

(72) Inventor: **Ballou, Margaret McKenzie**
**7626 Savannah Drive**
**Indianapolis, Indianapolis 46217(US)**

(74) Representative: **Hudson, Christopher Mark et al**
**Erl Wood Manor**
**Windlesham Surrey GU20 6PH(GB)**

(54) **A DNA segment isolated from phage FP43 functions as an origin of replication in streptomyces and related genera.**

(57) The present invention provides DNA compounds comprising a phage FP43 origin of replication. The phage FP43 derived origin of replication is useful in the construction of recombinant DNA vectors having a broad host range among the Streptomyces and related genera.

EP 0 438 231 A2

# A DNA SEGMENT ISOLATED FROM PHAGE FP43 FUNCTIONS AS AN ORIGIN OF REPLICATION IN STREPTOMYCES AND RELATED GENERA

The genus Streptomyces are gram positive bacteria characterized by filament stability and spore formation on aerial mycelia.

Streptomyces possess relatively large genomes, are physiologically complex as evidenced by their cellular differentiation cycle, and are unrivaled in the biosynthesis of useful products. Waksman's isolation of actinomycin in 1940 and streptomycin in 1943 (see Waksman's "The Actinomycetes", Vols. 1, 2 and 3. 1962, Williams and Wilkins, Baltimore), launched an era of research devoted to the discovery of microbial products of medicinal and industrial utility. The Streptomyces have yielded over 500 different useful compounds including most antibiotics of practical use. Included in the repertoire of Streptomyces derived commercial products are the following: streptozocin, S. achromogenes, NRRL 3125, Singaran, et al., 1979, J. Antibiotics 32:379-385; spiramycin, S. ambofaciens, ATCC 15154, Omura and Nakagawa, 1975, J. Antibiotics 28:401-433; narasin, S. aureofaciens, NRRL 8092, Boeck, et al., 1977, Dev. Ind. Microbiology 18471-485; monensin, S. cinnamonensis, ATCC 15413, Day, et al., 1973, Antimicrob. Agents Chemotherapy 4:410-444; cirramycin, S. cirratus, ATCC 21731, Omura and Nakagawa, 1975, J. Antibiotics 28:401-433; methylenomycin and actinorhodin, S, coelicolor A3, Dowding, 1973, J. Gen. Microbiology 76:163-176 and Hopwood, 1967, Bacteriological Reviews 31:373-403; erythromycin, S, erythreus, NRRL 2338, Yi-guang, et al., 1982, J. Antibiotics 35:335-342; angolamycin, S. eurythermus, ATCC 14975, Omura and Nakagawa, 1975, J. Antibiotics 28:401-43; tylosin, S. fradiae, T59235, Seno and Baltz, 1981, Antimicrob. Agents Chemotherapy 20:370-377; S. fungicidus, ATCC 21574, Omura and Nakagawa, 1975, J. Antibiotics 28:401-433; various B-lactam antibiotics, S. lipmanii, ATCC 27357, Nagarajan, et al., 1971, J. Am. Chem. Soc. 193-2308-2310; carbomycin, S. macrosporeus, ATCC 19782, Omura and Nakagawa, 1975, J. Antibiotics 28:401-433; epsinomycins, S. mycarafaciens, ATCC 21454, Omura and Nakagawa, 1975, J. Antibiotics 28:401-433; and platenomycins, S. platensis, ATCC 29778, Omura and Nakagawa, 1975, J. Antibiotics 28:401-433; and chloramphenicol, S. venezulae, ATCC 10712, Stuttard and Dwyer, 1981, Can. J. Microbiology 27:496-499 and Stuttard, 1979, J. Gen. Microbiology 110:479-482.

In the interest of brevity, the preceding list consisted of antimicrobial and anti-tumor products, but it should be noted that Streptomycetes are also well known for their role in soil from which most known species were isolated. Streptomyces break down proteins, cellulose, and other organic matters even including paraffin, probably contributing as much as all other bacteria and fungi to soil fertility and to the geochemical stability of the biosphere.

The ability to subjugate a genus of this clinical, industrial, and agricultural import has been impeded by their refractiveness to the manipulations of genetic engineering. The recent developments of phage isolation, protoplast fusion, and plasmid transformation of protoplasts are allowing progress in the genetic engineering of Streptomyces.

Polyethyleneglycol (PEG) induced plasmid transformation of protoplasts has allowed the development of gene cloning procedures for several species of Streptomyces and related genera. Thus, Acebal et al., 1986, FEMS Microbiol. Lett. 35:79-82, describe a method for transforming S. wadayamensis, a β-lactam antibiotic producer; Bibb et al., 1978, Nature 274:398-400 describe high frequency transformation of plasmid DNA into Streptomyces; Lampel and Strohl, 1986, Appl. Environ. Microbiol. 51:126-131, describe transformation and transfection of anthracycline-producing streptomycetes; Matsushima and Baltz, 1985, J. Bacteriol. 163:180-185, describe efficient plasmid transformation of S. ambofaciens and S. fradiae; Yamanoto et al., 1986, J. Antibiot. 39:1304-1313, describe transformation of S. erythraceus; Pidcock, et al., 1985, Appl. Environ. Microbiol. 50:693-695, describe transformation of Thermomonospora fusca protoplasts.

Further progress in the genetic engineering of Streptomyces appears only to be limited by the availability of gene expression systems which would be of utility in many related Streptomyces and the refinement of transformation and transduction systems for the efficient introduction of these expression systems.

Surprisingly, a DNA sequence isolated from bacteriophage FP43 provides an origin of replication, which functions in Streptomyces and related genera and thus allows construction of recombinant DNA vectors useful in the genetic engineering of Streptomyces and related genera.

Figures are provided to illustrate the construction of recombinant DNA vectors comprising the FP43 origin of Replication. The figures described below are drawn to scale; however, observed restriction fragment size may vary somewhat from calculated size based on map distance. For some restriction enzymes, only certain cut sites are shown for convenience.

Figure 1 is a restriction map of the ~4.3 kb SphI fragment isolated from phage FP43.

Figure 2 is a restriction site and function map of plasmid pKC684.

Figure 3 is a restriction site and function map of plasmid pKC685.

Figure 4 is a restriction site and function map of plasmid pKC644.

Figure 5 is a restriction site and function map of the 32kb MboI fragment isolated from the Streptomyces ambofaciens genome.

Figure 6 is a restriction site and function map of plasmid pKC702.

Figure 7 is a restriction site and function map of plasmid pKC703.

Figure 8 is a restriction site and function map of plasmid pKC721.

Figure 9 is a restriction site and function map of plasmid pKC761.

Figure 10 is a restriction site and function map of plasmid pKC978.

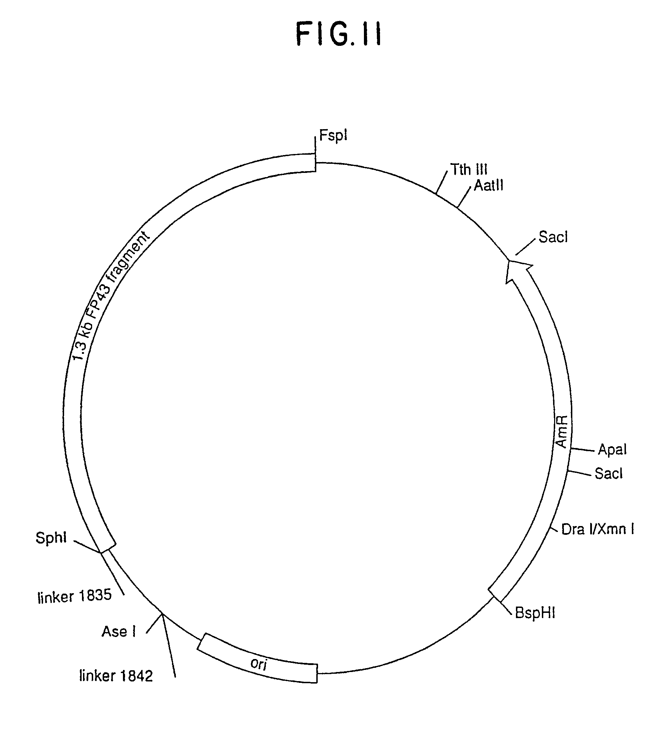

Figure 11 is a restriction site and function map of plasmid pKC1062.

The present invention discloses a segment of DNA isolated from the Streptomyces phage FP43 which functions as an origin of replication in Streptomyces and related genera. Additionally, the present invention provides recombinant DNA vectors, which replicate freely in a Streptomyces or a related host, and by virtue of their derivation from phage are compatible with other plasmids. Several Streptomyces species have been transformed with recombinant DNA vectors containing the FP43 replicon and plasmids which also contain an E. coli origin of replication have demonstrated utility as shuttle vectors. The origin of replication disclosed in this invention resides on an ~1.3 kb fragment of phage FP43. Instances in which the size of vector DNA is critical are common and the small size of this invention allows insertion of larger regulatory and coding sequences for useful substances. The broad range of Streptomyces species successfully transformed by the invention; the instability of expression vectors containing the origin of the invention in the absence of antibiotic selection; the small portion of total vector size required by the invention, and the utility as a shuttle vector among the Streptomycetes species and other genera of bacteria all support this invention as an advancement in the genetic engineering of Streptomyces and related genera.

The present invention provides DNA compounds comprising an origin of replication isolated from phage FP43. Phage FP43 is a temperate bacteriophage with a broad host range. The host range of phage FP43 comprises Streptomyces and related genera. (See Cox and Baltz, 1984, J. Bacteriol. 159:499-504). Phage FP43's host range also includes several genera related to Streptomyces including Chainia, Saccharopolyspora, and Streptoverticillium. See European Patent Application No. 88301763.4, published September 7, 1988. An origin of replication derived from phage FP43 (FP43 replicon) is especially useful in the construction of recombinant DNA vectors for use in Streptomyces and related genera.

Amplification and manipulation of plasmids is done faster and more efficiently in E. coli than in Streptomyces. It is convenient to add DNA sequences that allow for replication in E. coli. Thus, the additions of functional replicon-containing or antibiotic-resistance-conferring restriction fragments from E. coli vectors such as for example pBR322, pACYC184, pUC19 and the like are highly advantageous and add to the general utility of the present vectors.

The vectors of the present invention are known to contain multiple restriction endonuclease sites. Further restriction sites in the form of multiple cloning site cassettes can conveniently be added to further the general utility of the recombinant DNA vectors of the invention.

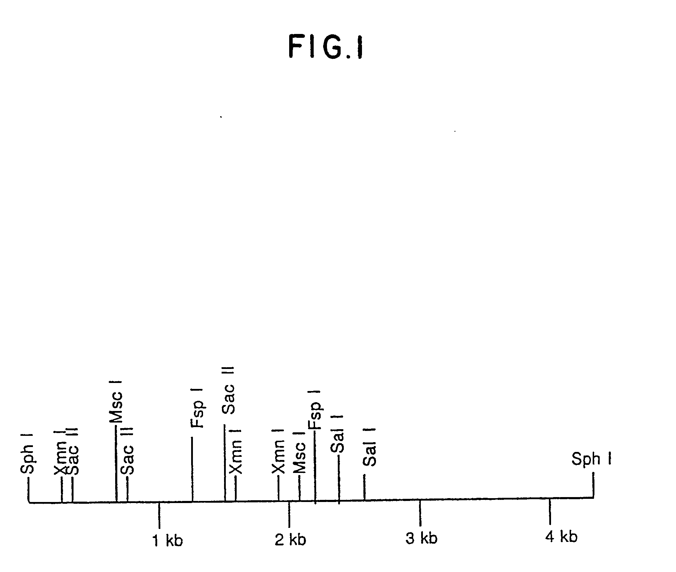

Streptomyces griseofuscus (FP43) contains lysogenized phage FP43 and is available from the Agricultural Research Service, Northern Regional Research Center (NRRL), Peoria, IL 61604, under the accession number NRRL 18184. The origin of replication of this invention was mapped to the ~4.3 kb SphI fragment of phage FP43. A restriction endonuclease map of the ~4.3 kb sequence of phage FP43, which comprises a phage FP43 origin of replication is provided in Figure 1.

Example 1 teaches the isolation of phage FP43 from S. griseofuscus (FP43) NRRL 18184. Thus, skilled artisans guided by the teachings of Example 1 and Figure 1 can isolate the DNA sequences of the present invention by purifying the appropriate restriction endonuclease fragments of phage FP43.

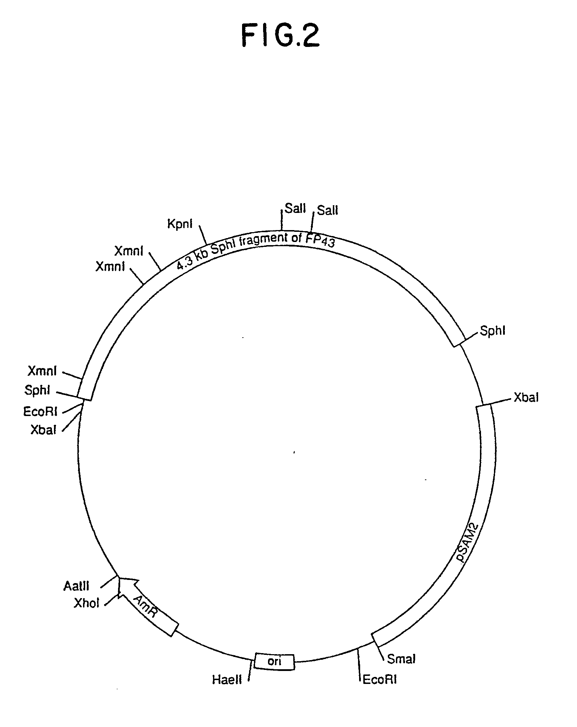

The preferred source of the DNA compounds of the present invention is plasmid pKC684. Plasmid pKC684 has been deposited with the NRRL and is available under the accession number, NRRL B-18541. A restriction site and function map of plasmid pKC684 is provided in Figure 2. Plasmid pKC684 comprises an ~4.3 kb SphI fragment derived from phage FP43, an ~2.9 kb fragment comprising a plasmid pUC derived E. coli origin of replication, an apramycin resistance marker, and an ~2.8 kb fragment derived from the resident S. ambofaciens plasmid pSAM2. Plasmid pKC684 replicates in E. coli and is readily selectable by virtue of the apramycin resistance marker. Cultivation in E. coli is preferred as a source of the DNA compounds of the invention. Plasmid pKC684 replicates in Streptomyces griseofuscus; however the plasmid pSAM2 derived sequence effects integration of the plasmid pKC684 into the Streptomyces chromosome. Thus, cultivation of plasmid pKC684 in E. coli host cells provides a more convenient means for preparing plasmid pKC684.

Plasmid pKC684 and plasmid pKC685 differ only in the orientation of the E. coli origin and apramycin resistance marker comprising the ~2.9 kb EcoRI fragment. The preparation of plasmid pKC685 is taught in Example 3. The illustrative plasmids pKC684 and pKC685 both comprise an origin of replication which is functional in Streptomyces and related genera.

The preparation of total DNA and plasmid DNA from Streptomyces transformants (Example 7) and the analysis of said DNA by Southern-blot analysis (Example 11) were utilized to determine whether the illustrative recombinant DNA vectors of the invention were integrated or were replicating autonomously.

An important embodiment of the recombinant DNA vectors of the present invention is illustrated by plasmids pKC684 and pKC685. Recombinant DNA vectors comprising the plasmid pKC684 or pKC685 replicate within the Streptomyces host cell. The FP43 replicon allows amplification of the genes cloned into the illustrative plasmids pKC684 or pKC685 prior to integration of the plasmids into the Streptomyces chromosome via the pSAM2 sequences of plasmids pKC684 and pKC685. Thus, genes cloned into plasmids may be amplified by plasmid replication prior to integration into the host cell chromosome. This ability to increase the number of genes integrated into the Streptomyces chromosome is useful when the genes being inserted are for biosynthetic complementation.

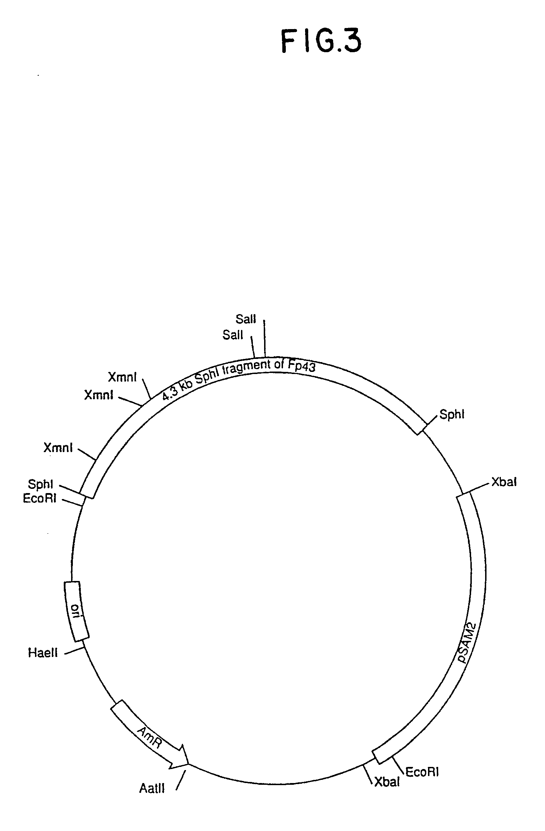

Biosynthetic complementation allows for more efficient production of antibiotics or other useful secondary metabolites by inserting additional copies of genes which comprise the rate-limiting steps of enzymes in biosynthetic pathways. The restriction site and function maps of plasmids pKC684 and pKC685 provided in Figures 2 and 3 respectively, enable the skilled artisan to clone biosynthetic pathway genes into illustrative plasmids pKC684 and pKC685 to produce recombinant DNA vectors, which upon transformation into Streptomyces or related genera will replicate, thereby increasing the number of the biosynthetic pathway genes present in the cell. The integration sequence derived from plasmid pSAM2 and provided by plasmids pKC684 and pKC685 then functions to insert the recombinant DNA cloning vector into the host cell chromosome and provide a stable deposition of the vector even in the absence of selective pressure.

The biosynthetic pathway genes for several antibiotics are well known. Because the FP43 replicon functions in a broad range of antibiotic producing organisms, recombinant DNA cloning vectors comprising the FP43 replicon and antibiotic biosynthetic pathway genes are especially useful for biosynthetic complementation to improve the yields of known antibiotics by providing additional gene products for rate-limiting steps as well as to generate novel antibiotics through the biosynthetic intercourse of differing biosynthetic pathways. The ability to produce hybrid macrolide antibiotics by combining various macrolide biosynthetic pathways, due to the "loose" substrate specificities of some of the biosynthetic enzymes (Sadakane et al., 1982, J. Antibiotics 35:680-687) is illustrative of the utility of this aspect of the present invention.

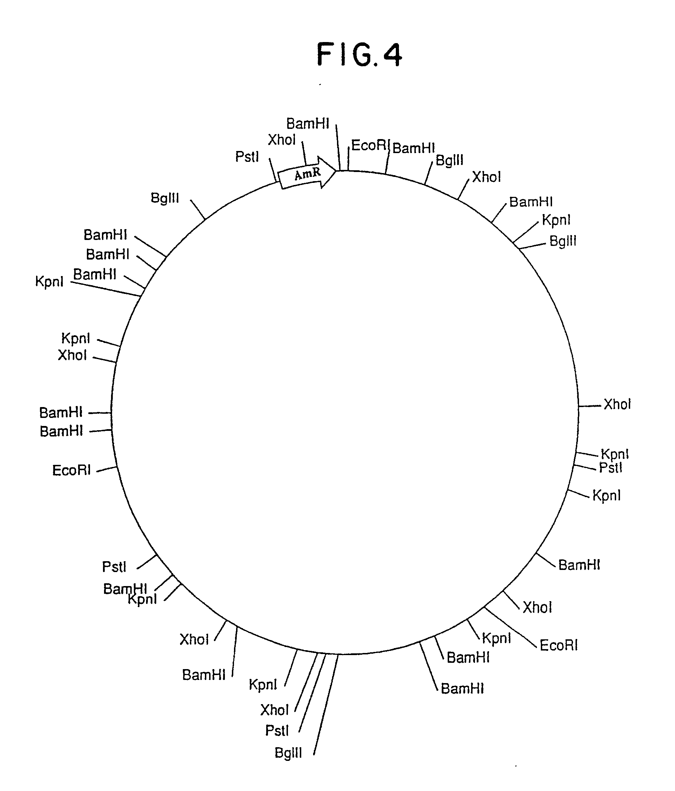

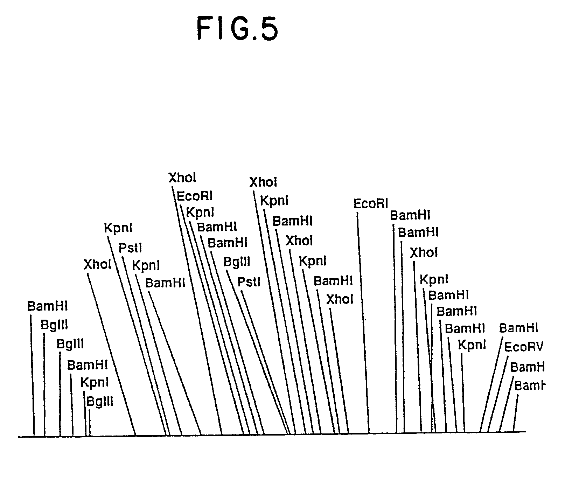

Genes encoding 5 spiramycin antibiotic biosynthetic genes are located on a span of ~32 kb of the Streptomyces ambofaciens genome. The cosmid pKC644 has insert DNA which comprises this ~32 kb span. Cosmid pKC644 has been deposited in the NRRL as E. coli K12 DK22/pKC644 and is available under the accession number NRRL B-18238. A restriction site and function map of cosmid pKC644 is provided in Figure 4. The location of the genes on the ~32 kb Mbo I fragment isolated from the S. ambofaciens genome and provided in cosmid pKC644 is shown in Figure 5. Those skilled in the art will recognize appropriate methods for cloning the biosynthetic pathway genes provided in cosmid pKC644 into the vectors of the present invention. The broad host range of the FP43 replicon and the integration function of the plasmid pSAM2 insert make the recombinant DNA cloning vectors of the present invention especially useful for shuttling biosynthetic genes into Streptomyces and related genera.

Further illustrative examples of biosynthetic pathway genes which can be cloned into the recombinant DNA vectors of the present invention to construct further recombinant DNA vectors of the invention include, but are not limited to: cephalosporins, actaplanin, apramycin, narasin, monensin, tobramycin, tylosin, erythromycin and the like.

An important aspect of this embodiment of the present invention is that because the shuttled antibiotic biosynthetic genes are integrated into the host cell chromosome, the need for antibiotic selection to maintain an extra-chromosomal element is eliminated. The presence of the apramycin resistance gene on the integrated vectors as well as the Southern-blot analysis described in Example 11 provides a convenient means for confirming the presence of the integrated DNA.

Plasmids pKC702 and plasmid pKC703 were derived from plasmids pKC684 and pKC685 respectively by deletion of the plasmid pSAM2 derived insertion sequence in the case of pKC703 and deletion of the phage FP43 derived insertion sequence in the case of pKC702. Construction of plasmids pKC702 and pKC703 is taught in Examples 4 and 5 respectively. The deletion of the plasmid pSAM2 sequence allows transformation of Streptomyces with plasmid pKC703 without the subsequent integration into the host cell chromosome. Plasmid pKC703 is readily prepared in either Streptomyces or E. coli. Example 7 teaches

4

methods for Streptomyces protoplast preparation, Streptomyces transformation, cultivation of Streptomyces, and plasmid isolation from Streptomyces. Transformation of Streptomyces with plasmid pKC703 illustrates the functionality of the phage FP43 derived origin of replication. Transformation of E. coli with plasmids pKC702 and pKC703 are the preferred methods for preparing plasmid pKC702 and pKC703 DNA because of the ease with which E. coli are transformed and cultivated, and the ease with which plasmids are prepared from E. coli. E. coli DH5∝ (Bethesda Research Laboratories P.O. Box 577, Gaithersburg, MD 20760 (BRL) and E. coli XL-1 Blue (Stratagene, 3770 Tansy St., San Diego, CA 92121) were used in all transformation procedures utilizing E. coli. As a matter of convenience, it is preferred to purchase the E. coli as competent cultures. Both the E. coli DH5∝ and E. coli XL-1 Blue are preferred strains of E. coli for the purposes of the present invention.

Plasmid pKC703 contains a phage FP43 replicon and thus is illustrative of the numerous plasmids which can be prepared utilizing the DNA compounds of the present invention. The ~4.3 kb size of the phage FP43 replicon limits the size of additional DNA sequences, which can be incorporated into a recombinant DNA vector without disrupting the ability to transform the vectors into Streptomyces and related genera.

The smaller size of the FP43 derived origin of replication in plasmid pKC721 is convenient. Plasmid pKC721 was prepared by excising the ~2 kb Sal I fragment from plasmid pKC703. The resultant ~2.4 kb phage FP43 derived origin of replication is functional in Streptomyces. Transformation of Streptomyces griseofuscus with plasmid pKC721 resulted in replication of plasmid pKC721 in S. griseofuscus. The preparation of total DNA and plasmid DNA as taught in Example 7 and the Southern-blot analysis taught in Example 11 was used to confirm the replicative autonomy of plasmid pKC721 in S. griseofuscus.

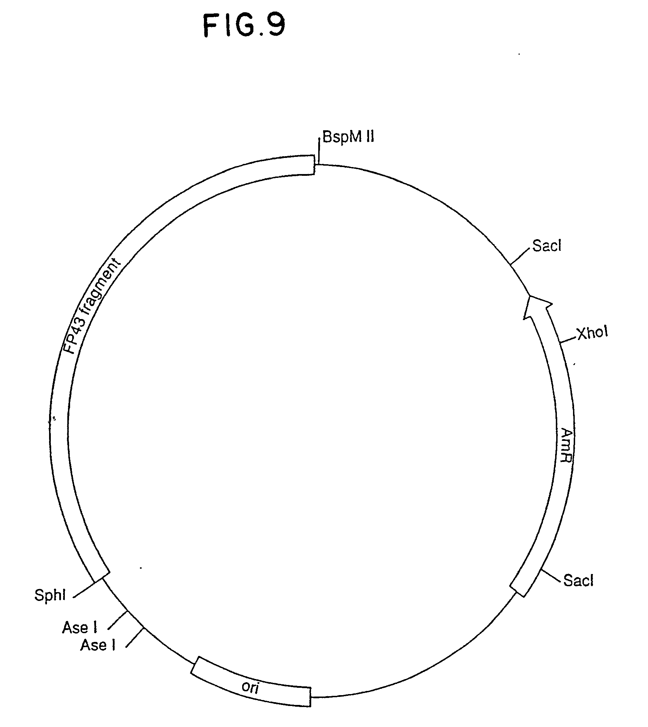

The size of the phage FP43 replicon was reduced to ~1.3 kb upon construction of plasmid pKC761. Construction of plasmid pKC761 is described in Example 9. A restriction site and function map of plasmid pKC761 is provided in Figure 9. Plasmid pKC761 was constructed by excising the ~1.5 kb Fsp I fragment from plasmid pKC721. The small ~1.3 kb FP43 replicon of plasmid pKC761 is a particularly convenient aspect of the DNA compounds comprising the phage FP43 origin of replication.

Recombinant DNA vectors comprising the ~1.3 kb phage FP43 replicon are useful for introducing DNA sequences such as biosynthetic genes into the chromosomes of Streptomyces and related genera. The utility of the vectors of the present invention to exploit the production of useful substances in Streptomyces and related genera was extended beyond the ability to amplify DNA for site specific integration into the chromosome. Cloning genes into illustrative plasmids pKC684 and plasmid pKC685 allows only the positioning of the cloned sequences at the pSAM2 insertion site.

Plasmid pKC978 is another illustrative plasmid of the present invention. Plasmid pKC978 comprises the ~1.3 kb FP43 replicon as its only means for automomous replication within Streptomyces and related genera, a spectinomycin resistance marker (SpcR), and an E. coli replicon derived from plasmid pACYC 184. Thus, plasmid pKC978 illustrates further utilities of the FP43 replicon. Plasmid pKC978 is an illustrative plasmid which due to the pACYC 184 derived replicon can coexist with plasmids which may be incompatible with other plasmids of the invention. The spectinomycin resistance marker provides a convenient method for selecting host cells comprising pKC978.

Illustrative plasmids pKC761 and pKC1062 are useful for introducing DNA sequences which integrate into the chromosome of Streptomyces and related genera through homologous recombination. The spiramycin biosynthetic genes of Figure 5 are merely illustrative of genes which can be cloned into illustrative plasmids pKC761 and pKC1062, transformed into host cells, and allowed to integrate into the chromosome via homologous recombination. The ability to introduce such genes into a wide range of host cells significantly extends the art of Streptomyces genetics. Genes or gene clusters can be cloned, mutagenized, and reinserted into the host cell chromosome to map biosynthetic pathways, or improve production yields by replacement of inferior biosynthetic enzymes with more efficient enzymes, as well as introduce novel enzymes into biosynthetic pathways for the production of novel antibiotics or other useful secondary metabolites.

Recombinant DNA vectors of the invention are especially useful for introducing genes into the chromosomes of Streptomyces and related genera. When the antibiotic used as a selection marker is removed from host cells transformed with the illustrative autonomously replicating recombinant DNA vectors of the present invention, the vectors are lost by more than 99% of the transformed cells. Thus, the instability of the vectors of the present invention facilitates the manipulation and utilization of DNA sequences inserted into the host cell chromosome without the problems attendant on also having unintegrated vectors present in the host cell. The ~1.3 kb FP43 replicon can be further reduced in size by skills well known in the art. A restriction map of the ~4.3 kb Sph I fragment of phage FP43 is provided in Figure 1. Thus, the teachings of the present invention enable the derivation of sequences, smaller than ~1.3 kb, which function as a replicon.

The following examples further illustrate and describe the invention disclosed herein. The invention is not limited in scope by reason of any of the following examples; sources of reagents or equipment are provided merely for convenience and in no way limit the invention. Both an explanation of and the actual procedure are provided where appropriate.

Example 1

## Isolation of Phage FP43 from _Streptomyces griseofuscus_ C581 (FP43)

A. List of Solutions

The following solutions are referred to throughout the Examples and are presented here for clarity.

1. P Medium (~100 ml):

| Ingredient | Amount |
|---|---|
| Sucrose | 10.3 g |
| $K_2SO_4$ | 0.025 g |
| Trace element solution (see #3) | 0.2 ml |
| $MgCl_2 \cdot 6H_2O$ | 0.203 g |
| Water | 80 ml |
| After autoclaving add: | |
| $KH_2PO_4$ (0.5%) | 1 ml |
| $CaCl_2 \cdot 2H_2O$ (3.68%) | 10 ml |
| (N-tris-(hydroxymethyl)-methyl-2-aminoethane sulphonic acid), "TES" buffer, 0.25 M, pH=7.2 | 10 ml |

2. Trace element solution (~1 L):

| Ingredient | Amount |
|---|---|
| $ZnCl_2$ | 40 mg |
| $FeCl_3 \cdot 6H_2O$ | 200 mg |
| $CuCl_2 \cdot 2H_2O$ | 10 mg |
| $MnCl_2 \cdot 4H_2O$ | 10 mg |
| $Na_2B_4O_7 \cdot 10H_2O$ | 10 mg |
| $(NH_4)_6Mo_7O_{24} \cdot 4H_2O$ | 10 mg |
| $H_2O$ | 1 L |

3. R2 Medium (~1 L):

| Ingredient | Amount |
|---|---|
| Sucrose | 103 g |
| $K_2SO_4$ | 0.25 g |
| Trace element solution | 2 ml |
| $MgCl_2 \cdot 6H_2O$ | 10.12 g |
| glucose | 10 g |
| L-asparagine $\cdot 1H_2O$ | 2.0 g |
| casamino acids | 0.1 g |
| Agar | 22 g |
| Water | to 700 ml |

The pH is adjusted to pH = 7.2 before autoclaving. After autoclaving, add:

| | |
|---|---|
| $KH_2PO_4$ (0.05 g/100 ml) | 100 ml |
| $CaCl_2$ (2.22 g/100 ml) | 100 ml |
| TES Buffer (5.73 g/100 ml, pH = 7.2) | 100 ml |

R2 Soft Agar

| Ingredient | Amount |
|---|---|
| Sucrose | 103 g |
| $K_2SO_4$ | 0.25 g |
| $NgCl_2$ - 6 $H_2O$ | 10.12 g |
| Agar | 7 g |
| Water | to 800 ml |

The pH is adjusted to pH = 7.2 before autoclaving After autoclaving add:

| | |
|---|---|
| $CaCl_2$ (2.22 g/100 ml) | 100 ml |
| TES Buffer (5.73 g/100 ml, pH = 7.2) | 100 ml |

4. Soft Nutrient Agar (SNA, ~1 L):

| Ingredient | Amount |
|---|---|
| Difco Nutrient Broth | 8 g |
| Agar | 5 g |

5. R2YE medium is R2 medium with 20 ml of 25% yeast extract added per liter.
6. Yeast Extract - Malt Extract (YEME, ~1 L):

| Ingredient | Amount |
| --- | --- |
| Yeast extract | 3 g |
| Peptone | 5 g |
| Malt extract | 3 g |
| Glucose | 10 g |

7. YEME + 34% Sucrose Liquid complete Medium is YEME with 340 g/L of sucrose.

8. YMX Media (~1 L):

| Ingredient | Amount |
| --- | --- |
| Yeast extract | 3 g |
| Malt extract | 3 g |
| Glucose | 2 g |
| Agar | 20 g |

9. YMX Agar is 0.3% yeast extract, 0.3% malt extract, 0.2% dextrose, and 2.0% agar.

10. CSI Medium (~1 L):

| Ingredient | Amount |
| --- | --- |
| Soybean meal | 15 g |
| Casein | 1 g |
| Cerelose | 25 g |
| Blackstrap molasses | 3 g |
| $CaCO_3$ | 2.5 g |
| Czapek Mineral Stock | 2 ml |
| Water (deionized) | 1 L |
| pH adjusted to 7.2 prior to sterilization | |

11. Czapek's Mineral Mix (~1 L):

| | |
| --- | --- |
| KCl | 100 g |
| $MgSO_4 \cdot 7H_2O$ | 100 g |
| Deionized Water | 900 ml |

$FeSO_4 \cdot 7H_2O$ (2 g) was dissolved in 100 ml deionized water containing 2 ml of concentrated HCl. This solution was added to the above $KCl/MgSO_4 \cdot 7H_2O$ solution to complete preparation of the Czapek's Mineral Mix.

12. Bennett's Agar (~1 L):

| Ingredient | Amount |
| --- | --- |
| Deionized $H_2O$ | 1000 ml |
| Potato Dextrin | 10 g |
| N-Z Amine A | 2 g |
| *Gibco bactoagar | 15 g |
| Gibco beef extract | 2 g |
| Yeast extract | 1 g |
| Czapek's mineral stock | 2 ml |

*Gibco Laboratories, 3175 Staley Road, Grand Island, N.Y. 14072

13. NC Broth, also referred to as NCB
NC broth contains 8 g of Difco (P.O. Box 1058, Detroit, MI 48232) nutrient broth per liter of deionized $H_2O$ and is also 4 mM in $Ca(NO_3)_2$.

14. TES Buffer
TES is an abbreviation for 2-{(tris-[hydroxymethyl]-methyl)amino}ethanesulfonic; to prepare TES buffer, a 1 M solution of TES acid (125.6 g/500 ml) is mixed with a one-half volume of 1 M TES base and then diluted with distilled water to 0.25 M in TES.

15. Sevag
Sevag is a 24:1 mixture of chloroform:isoamyl alcohol

16. ø Buffer
ø buffer is 10 mM TES and 10 mM $Ca(NO_3)_2$.

17. TE Buffer
TE buffer contains 10 mM Tris-HCl, pH = 8, and 1 mM $Na_2EDTA$.

18. R2 Overlays (per 1 L)

| Ingredient | Amount |
| --- | --- |
| Sucrose | 103 g |
| $MgCl_2$ | 10.12 g |
| 0.151 M $CaCl_2$ | 100 ml |
| TES Buffer | 100 ml |
| Gibco Agar | 4.1 g |
| Distilled $H_2O$ | to 1 L |

19. TSS Broth
TSS broth is prepared by adding 51.9 ml of 60% sucrose to 250 ml of tripicase soy broth (TSB). TSB is available from Baltimore Biological Laboratories, Inc. (BBL), P.O. Box 243, Cockeysville, MD 21031. As used herein, the TSS broth also contains enough glycine to give a final glycine concentration of 0.5%.

20. Tris is an abbreviation for Tris(hydroxymethyl) aminomethane. Tris buffers are well known in the art and are commercially available from Sigma Chemical Co., P.O. Box 14508, St. Louis, MO 63178.

21. NuSieve GTG agarose is a low melting point agarose which is commercially available from FMC Corp., Marine Colloids Division, Rockland, ME 04841.

22. EDTA is an abbreviation for ethylenediaminetetraacetic acid. EDTA is available from the Sigma Chemical Co.

B. Procedures The procedures described below are well known to the skilled artisan. Details and references to procedures are provided merely to further convenience those who wish to practice the present invention.

(1) Centrifugation, unless otherwise specified in the examples, refers to a five minute centrifugation in an Eppendorf table-top centrifuge at room temperature. The centrifugal force generated therein is approximately 15,000 xg.

(2) Agarose gel electrophoresis is a technique well known in art. Agarose gel electrophoresis is described in great detail in T. Maniatis, E. Fritsch, and J. Sambrook, Molecular Cloning-A Laboratory Manual (1982), hereinafter Maniatis, at 150-171. Recovery of DNA bands from low melting point agarose such as NuSieve GTG is described in Maniatis at p. 170.

(3) Large scale isolation of plasmid DNA is described in Maniatis at pages 86-94. Plasmid purifications on cesium chloride-ethidium bromide gradients are described at pages 93-94.

(4) Cultivation of micro-organisms is well known in the art of microbiology. D. Hopwood, et al., Genetic Manipulations of Streptomyces-A Laboratory Manual (1985) is an excellent reference manual for all cultivation procedures and genetic engineering considerations.

B. Phage Isolation

Phage lysates and DNA were prepared in substantial accordance with the procedure described in Cox and Baltz, 1984, J. Bacteriol. 159: 499-504. A lyophilized culture of Streptomyces griseofuscus C581(FP43) is obtained from the Northern Regional Research Center (NRRL), Agricultural Research Service, Peoria, IL 61604 under the accession number NRRL 18184. The lyophilized culture is used to inoculate 10 ml of NC broth; the culture is then incubated at 29°C in a gyratory incubator overnight (~16 hours). The culture is centrifuged to remove the cells and cellular debris; the supernatant is then passed through a 0.45 $\mu$ filter, and the filtrate is saved as it contains phage FP43 particles.

A lyophil of Streptomyces griseofuscus C581 is obtained from the American Type Culture Collection (ATCC), Rockville, MD 20852 under the accession number ATCC 23916. The lyophilized culture is used to inoculate 10 ml of TSB broth (BBL), in a 50 ml flask. The culture is incubated at 29°C in a gyratory incubator overnight.

Four 100 $\mu$l aliquots of the overnight culture of Streptomyces griseofuscus C581 are prepared and mixed, respectively, with 1.0 ml, 100 $\mu$l, 10 $\mu$l, and 1 $\mu$l of the lysate solution. The mixtures are then individually plated on NC agar (NC broth with 15 g/L agar) in 100 x 15 mm Petri plates and incubated at 34°C overnight. The following morning, the plates are examined, and the plate showing nearly confluent lysis is used to prepare the phage FP43 stock solution. The FP43 stock solution is prepared by adding ~5 ml of NC broth to the plate showing nearly confluent lysis, incubating the plate at room temperature for one to two hours, and collecting the broth from the plate. The solution is centrifuged and the resulting supernatant passed through a 0.45 $\mu$ filter to remove debris. The resulting phage FP43 solution typically contains $10^8$ to $10^{10}$ FP43 particles per ml--the exact titer is determined by plating several dilutions of the phage stock on a sensitive strain, such as S. griseofuscus C581.

C. Phage DNA Isolation

To prepare phage FP43 DNA, the procedure described in Example 1B was followed, except the lysates were prepared using four to six 9.5" x 9.5" Petri dishes. The plates were washed with 50 ml of NC broth to collect the phage particles. The lysates were centrifuged and the supernatants passed through a 0.45 $\mu$ filter to remove cellular debris. The lysate was then centrifuged for 2 hours at 25°C at 30,000 rpm to pellet the phage particles. Each pellet was resuspended in 1 ml of ø buffer; this solution was centrifuged in a tabletop centrifuge to pellet material that did not go back into solution. The supernatant was then layered on top of a CsCl gradient composed as follows (from most dense to least dense): 750 $\mu$l of 1.7 p CsCl in ø buffer; 750 $\mu$l of 1.6 p CsCl in ø buffer; 750 $\mu$l of 1.4 p CsCl in ø buffer; and ~2.45 ml of phage FP43 in ø buffer. The gradient was prepared in a polyallomer tube ($\frac{1}{2}$" x $\frac{1}{2}$"), which was placed in an SW50.1 rotor (Beckman Instruments, Inc., Spinco Division, P.O. Box 10200, Palo Alto, CA 94304). The solution was centrifuged at 25,000 rpm at 15°C for 1 hour and yielded two bands: a brown-tinged top band and a blue-tinged lower band. The lower band was collected with a syringe and dialyzed against 2 to 3 liters of ø buffer overnight.

The dialyzed band was extracted for 30 minutes with 1.5 volumes of ø buffer-saturated phenol (25 ml phenol:10 ml ø buffer) and then re-extracted for 10 minutes with another 1.5 volumes of ø buffer-saturated phenol. The phage band was then extracted 3 times with one volume of Sevag. The phage DNA was precipitated with 0.1 volume of 3 M sodium acetate (NaOAc), pH = 8.0, and 1 volume of isopropanol. The precipitated phage FP43 DNA was spooled from the solution, washed with 70% ethanol and resuspended in ~1 ml of TE buffer, yielding a solution containing ~0.5 mg/ml of phage FP43 DNA.

Example 2

A. Culture of E. coli DH5∝/pKC684

A lyophil of E. coli DH5∝/pKC684 can be obtained from the Northern Regional Research Laboratories (NRRL), Peoria, IL 61604, under the accession number B-18541 (date of deposit: 10/27/89) and used directly as the "culture" in the process described below.

One liter of TY broth (10 g tryptone, 5 g NaCl, and 5 g yeast extract per liter) containing 100 $\mu$g/ml apramycin is inoculated with a culture of E. coli DH5∝/pKC684 and incubated with aeration at 37° C overnight (15-18 hours). The resulting culture is used as a source of plasmid pKC684.

B. Isolation of Plasmid pKC684

The culture prepared in Example 2A is centrifuged at 8000 rpm using a GSA rotor for 10 minutes at 4° C to pellet the cells. The resulting supernatant is discarded. The cell pellet is resuspended in 28 ml of a solution containing 25% w/v sucrose and 50 mM Tris-HCl, pH = 8.0. The following are added to the resuspended cells: 1 ml of 5 mg/ml lysozyme (freshly prepared in the resuspension solution); 1.6 ml of 0.5 M EDTA (pH 8), and 0.2 ml of 5 mg/ml RNase A. The resulting mixture is incubated on ice for 15 minutes. Three ml of lysing solution (50 mM Tris-HCl, pH = 8.0, 3% Triton X-100 w/v, and 200 mM EDTA) are added to the lysozyme-treated cells with gentle mixing. The resulting solution is incubated on ice for another 15 minutes.

The cellular debris is removed from the solution by centrifugation at 20,000 rpm for about 45 minutes at 4° C. About 28.6 g of CsCl and ~1 ml of a 5 mg/ml ethidium bromide solution are added to the ~30 ml of supernatant. Then, the volume is adjusted to 40 ml with water and the solution decanted into an ultracentrifuge tube. The tube is sealed, and the solution is centrifuged at 49,000 rpm for ~18 hours. The resulting plasmid band, visualized with ultraviolet light, is isolated, extracted with 4 to 5 treatments with isobutanol to remove the ethidium bromide, and dialysed against three changes of ~20 volumes of TE buffer (10 mM Tris-HCl, pH = 7.5, and 1 mM EDTA). The dialysate is collected; then extracted with 2 treatments of equal volumes of phenol (equilibrated with TE) ~pH 8 followed by 2 equal volume Sevag extractions. Three volumes of ethanol and 0.1 volumes of 3 M sodium acetate solution are added to the aqueous extractions and the plasmid DNA is pelleted by centrifugation at 10,000 rpm using a sorvall HB4 rotor at -4° C. The resulting DNA pellet is rinsed first with 70% ethanol and then with 100% ethanol and dried under vacuum.

The ~1.0 mg of plasmid pKC684 DNA obtained by this procedure is suspended in 1.5 ml of 1X TE buffer and stored at 4° C. A restriction site and function map of plasmid pKC684 is presented in Figure 2 of the accompanying drawings.

Example 3

Plasmid pKC685

Plasmids pKC684 and pKC685 differ only in the orientation of the ~2.9 kb EcoRI fragment. Plasmid pKC685 can be prepared by digesting the plasmid pKC684 obtained in Example 2 with the restriction endonuclease EcoRI and ligating the phenol extracted digestion mixture.

(A) Construction of Plasmid pKC685

Three $\mu$g of plasmid pKC684 were digested at 37° C with ~10 U of EcoRI (obtained from New England Biolabs, 32 Trozer Road, Beverly, MA 01915-5510) in a digestion mixture comprising: 100mM NaCL, 100 mM Tris-HCl (pH = 7.5), 5 mM MgCl₂, 100 $\mu$g/ml bovine serum albumin, and 10mM DTT (dithiothreitol). (EcoRI digestion buffer).

The EcoRI digest of plasmid pKC684 was then ethanol precipitated, washed, and vacuum dried. The digestion components were then ligated using ~10 U T4 DNA ligase (New England Biolabs) in a final volume of 20 $\mu$l. The ligation buffer consisted of 50 mM Tris-HCl (pH = 7.8), 10 mM MgCl₂ 20 mM DTT, 1 mM ATP and 50 $\mu$g/ml bovine serum albumin. The ligation proceeded at ~16° C for about 18 hours.

(B) E. coli Transformation Protocol

The transformation protocol provided below was used in all aspects of the invention which involved E. coli transformation.

The ligation mixture is transformed into E. coli DH5∝ as follows. Preferably, the competent E. coli DH5∝ are purchased from Bethesda Research Lab, Gaithersburg, MD 20877. Alternatively, competent E. coli DH5∝ can be prepared in substantial accordance with the method of Maniatis, 1982 p. 252-253, the teachings of which are herein incorporated by reference. 2.5 $\mu$l of the ligation mixture is added to 100 $\mu$l of competent E. coli DH5∝ containing ~$10^8$ cells/ml. The transformation mixture is incubated on ice for approximately 30 minutes. The temperature is then raised by immersing the tube in a water bath at 42°C to "heat shock" the cells for approximately 1 minute. Cells are allowed to grow 2 hours in ~1 ml TY broth at 37°C on a roller drum and then selected by plating the transformation mixture on TY agar with apramycin (100 $\mu$g/ml).

Apramycin resistant colonies are then screened to determine which transformants comprise the plasmid pKC685. Plasmids are harvested from the transformed cells as follows.

## (C) Plasmid Preparation

The following protocol was used in all aspects of the invention, which involved isolation of plasmids from transformed E. coli.

Transformed E. coli DH5∝ were cultured overnight in 5 ml TY broth. 4 ml of the broth were then harvested for restriction analysis. Cells were pelleted by centrifugation. The cell-pellet was resuspended to a volume of approximately 500 $\mu$l in 25 mM Tris-HCl (pH = 8) with 25 mM EDTA. 250 $\mu$l of 0.2 N NaOH with 2% w/v SDS was added and the mixture was vortexed. 1.5 ml Eppendorf snap-top tubes are preferred containers for the plasmid preparations and all centrifugation steps were performed using a table-top Eppendorf centrifuge. The tubes were warmed to 70°C for ten minutes, then cooled to room temperature. 100 $\mu$l of phenol:chloroform (1:1) was added and the mixture was vortexed. The mixture was centrifuged ~3-5 minutes in a table-top Eppendorf centrifuge. The aqueous phase was aspirated and removed to a fresh 1.5 ml Eppendorf tube. 70 $\mu$l of 3 M sodium acetate were added, then the tube was filled with isopropanol. Following a 5-10 minute centrifugation the supernatant was decanted and the tube was centrifuged another 5 to 10 minutes. The remaining droplets were aspirated, leaving only the precipitated DNA in the tube. The DNA pellet was dissolved in 500 $\mu$l TE and 25 $\mu$l of 100 mM spermine was added. Centrifugation for 5 minutes at room temperature precipitated the DNA. The supernatant was decanted and the DNA pellet was then washed with Keiser wash solution, which comprises 300 $\mu$l of 0.3M sodium acetate with 100 mM $MgCl_2$ and 700 $\mu$l ethanol. After mixing the DNA and the Keiser wash solution, the DNA was centrifuged at room temperature for 5 minutes. The DNA pellet was then washed with ethanol, dried under vacuum and resuspended in 10-20 $\mu$l of TE.

## (D) Restriction Endonuclease Mapping

Plasmid DNA isolated from E. coli transformed with the ligation mixture of Example 3A was then analyzed for the orientation of the EcoRI insert to distinguish which transformants comprise pKC685, pKC684, or plasmids which had deleted the ~7.1 kb FP43 and pSAM$_2$ fragment. Digestion of plasmid pKC685 with Xba I produced ~7.2 kb and ~2.8 kb fragments. Digestion of plasmid pKC684 with Xba I produced ~5.5 kb and ~4.6 kb fragments. Xba I digestion of the deletion plasmid resulted in a single band of ~2.9 kb. ~3 $\mu$g of plasmid DNA was digested with ~10 U of Xba I for 1 hour at 37°C in 20 $\mu$l digestion buffer comprising 50 mM NaCl, 10 mM Tris-HCl (pH = 7.9), 10 mM $MgCl_2$, 10mM DTT and 100 $\mu$g/ml bovine serum albumin. (Xba I digestion buffer). Restriction patterns revealed by gel electrophoresis of the Xba I digests distinguished the orientation of the EcoRI fragments corresponding to pKC684 and pKC685.

## Example 4

### A. Construction of Plasmid pKC702

Plasmid pKC702 was derived from plasmid pKC684 by excision of the ~4.3 kb FP43 derived fragment. Approximately 3 $\mu$g of plasmid pKC684 was digested with 12 U Xmn I and 40 U Xba I in 20 $\mu$l of digestion buffer comprising 50 mM NaCl, 10 mM Tris-HCl (pH = 7.9), 10 mM $MgCl_2$, 10 mM DTT and 10 $\mu$g/ml bovine serum albumin, for 1 hour at 37°C. The pKC684 digest was then electrophoresed through 1% NuSieve GTG agarose in TAE (40 mM Tris-acetate, 1 mM EDTA pH 8.0). The 5.5 kb pKC684 fragment was excised by removing the band in an ~40$\mu$l gel plug. The gel plug containing the ~5.5 kb band of pKC684 was then heated to 70°C for 10 minutes.

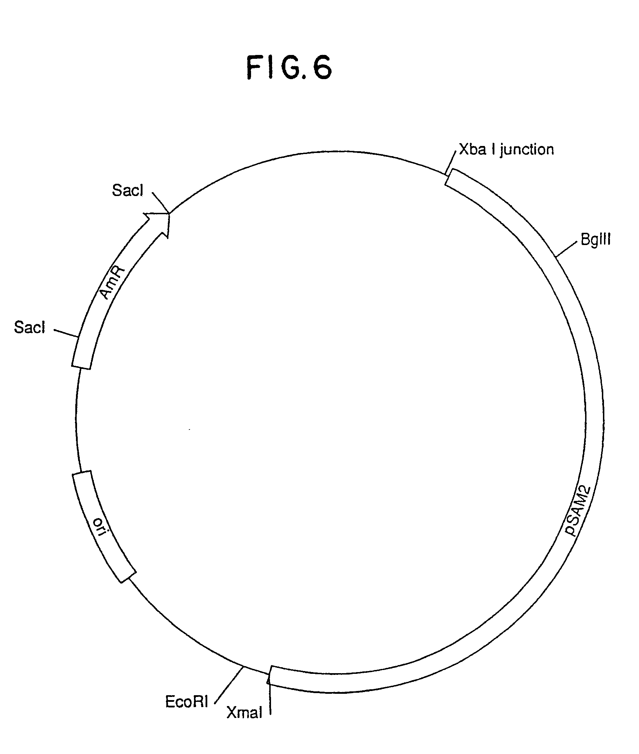

10 µl of the melted plug was added to a prewarmed, 37°C solution, comprising 2 µl 0.2M DTT, 2 µl 10X ligase buffer (500 mM Tris-HCl pH 7.8, 100 mM MgCl₂), 2 µl 10 mM ATP, 2 µl H₂O, and 2µl T4 DNA ligase (New England Biolabs). Ligation proceeded at 15°C overnight and resulted in plasmid pKC702. A restriction site and function map of pKC702 is provided in Figure 6.

B. Transformation of Competent E. Coli

The ligation mixture comprising plasmid pKC702 was used to transform competent E. coli DH5∝. 2.5 µl of the ligation mixture was warmed at 70°C for 5 minutes, then added to 100 µl of competent E. coli DH5∝. The transformation mixture was then incubated on ice for approximately 30 minutes. The cells were then heat shocked by a one minute incubation at 42°C. Cells were allowed to grow 2 hours in ~1 ml TY broth at 37°C on a roller drum and then selected by plating the transformation mixture on TY agar containing 100 µg/ml apramycin.

Apramycin resistant (AmR) clones were then screened to determine which transformants comprise plasmid pKC702. Screening was accomplished as follows. The transformants were cultured overnight at 37°C in separate tubes containing 5 ml of TY broth with apramycin (100 µg/ml).

Plasmids were harvested and prepared for restriction nuclease mapping in substantial accordance with the teachings of Example 3. 3 separate 1 µl samples were digested as follows. One 1 µl sample was digested with 10 U Bgl II (New England Biolabs) in a total volume of 20 µl in a digestion buffer consisting of 50 mM NaCl, 10 mM Tris-HCl (pH = 7.4), 10 mM MgCl₂, 10 mM DTT and 10 µl/ml bovine serum albumin (Bgl II digestion buffer), for 1 hour at 37°C. A second 1 µl sample of plasmid DNA was digested with 20 U of Hind III (New England Biolabs) for 1 hour at 37°C in a total volume of 20 µl. A digestion buffer consisting of 50 mM NaCl, 50 mM Tris-HCl (pH = 8.0), 10 mM MgCl₂, 10 mM DTT and 100 µg bovine serum albumin (Hind III digestion buffer) was used. A third 1 µl sample of plasmid DNA was digested with both Bgl II and Hind III in substantial accordance with the conditions set forth above. Gel electrophoresis of the digests and comparison of the fragment sizes to the restriction map of pKC702 were determinative of which transformants comprise plasmid pKC702.

Example 5

Construction of Plasmid pKC703

Plasmid pKC703 was prepared from plasmid pKC685 as follows. Plasmid pKC685 was prepared by culturing E. coli DH5∝/pKC685 and isolating plasmid DNA in substantial accordance with the method of Example 2A. 3 µl of plasmid pKC685 was digested with 20 U of Xba I (New England Biolabs) for 1 hour at 37°C in a 20 µl total volume of a digestion buffer comprising 50 mM NaCl, 10 mM Tris-HCl (pH = 7.9), 10 mM DTT, 10 mM MgCl₂, and 100 µg/ml bovine serum albumin (Xba I digestion buffer).

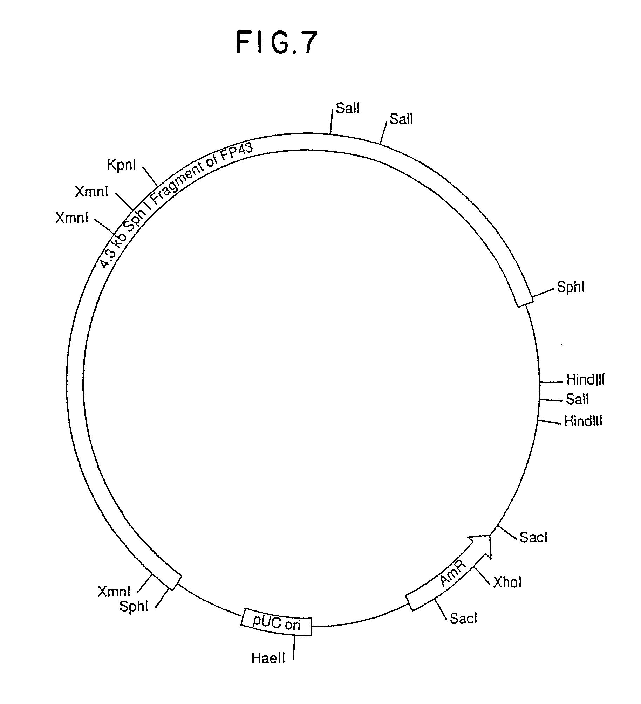

The Xba I digest of plasmid pKC685 was then separated by electrophoresis using 1% NuSieve GTG agarose in TAE buffer. The ~7.5 kb Xba I restriction fragment was harvested from the gel by removing the ~7.5 kb band in an ~40 µl plug. The gel plug containing the ~7.5 kb fragment was then melted and the ~7.5 kb fragment was ligated in substantial accord with the method of Example 4. Ligation of the ~7.5 kb Xba I fragment results in plasmid pKC703. A restriction site and function map of plasmid pKC703 is provided in Figure 7.

2.5 µl of the digestion mixture comprising plasmid pKC703 was used to transform competent E. coli DH5∝ (B.R.L.) in substantial accordance with the teaching of Example 4. Transformants were selected for apramycin resistance by plating the transformation mixture on TY agar containing apramycin (100 µg/ml). Isolated colonies were then screened for the presence of plasmid pKC703 as follows.

Transformed E. coli DH5∝ were cultured overnight in 5 ml TY broth. 4 ml of the broth was harvested for restriction analysis. Cells were pelleted by centrifugation. The cell-pellet was resuspended to a volume of approximately 500 µl in 25 mM Tris-HCl (pH = 8) with 25 mM EDTA. 250 µl of 0.2 N NaOH with 2% w/v SDS (sodium dodecyl sulfate) was added and the mixture was vortexed. 1.5 ml Eppendorf snap-top tubes are preferred containers for the plasmid preparations and all centrifugation steps were performed using a table-top Eppendorf centrifuge. The tubes were warmed to 70°C for ten minutes, then cooled to room temperature. 100 µl of phenol:chloroform (1:1) was added and the mixture was vortexed. The mixture was centrifuged ~3-5 minutes in a table-top Eppendorf centrifuge. The aqueous phase was harvested and removed to a fresh 1.5 ml Eppendorf tube. 70 µl of 3 M sodium acetate was added, then the tube was filled with isopropanol. Following a 5-10 minute centrifugation (table-top Eppendorf centrifuge), the supernatant was decanted and the tube was centrifuged another 5 to 10 minutes. The remaining droplets were aspirated, leaving only the precipitated DNA in the tube. The DNA pellet was dissolved in 500 $\mu$l TE and 25 $\mu$l of 100 mM spermine was then added. Centrifugation for 5 minutes at room temperature precipitated the DNA. The supernatant was decanted. The DNA pellet was then washed with Keiser wash solution. After mixing the DNA and the Keiser wash solution, the DNA was centrifuged at room temperature for 5 minutes. The DNA pellet was then washed with ethanol, dried under vacuum, and resuspended in 10-20 $\mu$l of TE.

The plasmid DNA prepared above was then mapped with the restriction endonucleases Bgl II, Hind III, and Xmn I. 1 $\mu$l of the plasmid preparation was digested with 8 U Bgl II (New England Biolabs) at 37°C for 1 hour in a total 20 $\mu$l digestion volume comprising 100 mM NaCl, 10 mM Tris-HCl (pH = 7.4), 10 mM MgCl$_2$, 10 mM DTT, and 100 $\mu$g/ml bovine serum albumin (Bgl II digestion buffer). A second 1 $\mu$l sample of the plasmid preparation was digested with 20 U Hind III (New England Biolabs) for 1 hour at 37°C in a total digestion volume of 20 $\mu$l comprising 50 mM NaCl, 50 mM Tris-HCl (pH = 8.0), 10 mM MgCl$_2$, and 10 U $\mu$g/ml bovine serum albumin (Hind III digestion buffer). A third 1 $\mu$l plasmid preparation sample was digested with 10 U of Xmn I (New England Biolabs) for 1 hour at 37°C in a 20 $\mu$l total digestion volume comprising 50 mM NaCl, 10 mM Tris-HCl (pH = 8.0), 10 mM MgCl$_2$, 10 mM DTT, and 100 $\mu$g/ml bovine serum albumin (Xmn I digestion buffer). Plasmid pKC703 was not cut by Bgl II, yielded a 7.5 kb fragment when digested with Hind III, and yielded fragments of ~5.5 kb, ~1.7 kb, and ~200 base pairs when digested with Xmn I.

Example 6

Construction of Plasmid pKC721

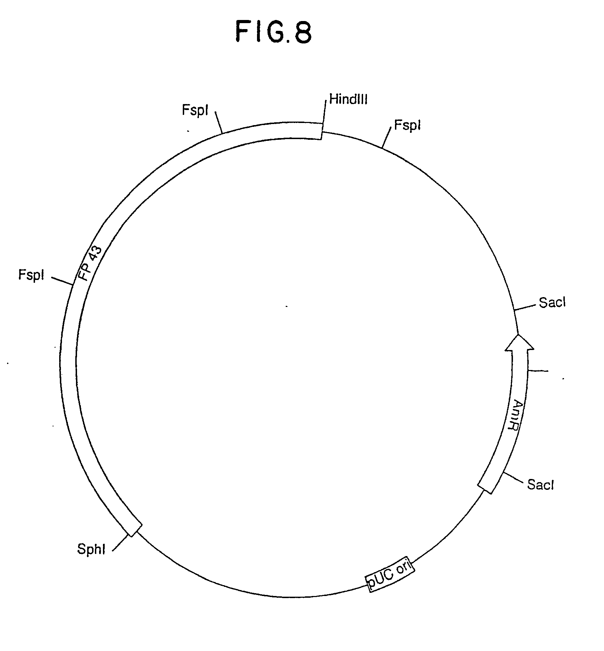

Plasmid pKC721 was constructed by deleting the ~2 kb Sal I fragment from plasmid pKC703. A restriction site and function map of plasmid pKC721 is provided in Figure 8.

Plasmid pKC703 DNA was prepared as taught in Example 5. 3$\mu$g of plasmid pKC703 was digested with 30 U of Sal I (New England Biolabs) in a 20 $\mu$l total digestion volume comprising: 150 mM NaCl, 10 mM Tris-HCl (pH = 7.9), 10 mM MgCl$_2$, 10 mM DTT, and 100 $\mu$g/ml bovine serum albumin (Sal I digestion buffer). The digestion mixture was then electrophoresed on a 1% NuSieve GTG agarose gel and the ~5.5 kb band removed therefrom in a 40 $\mu$l plug. The gel plug containing the ~5.5 kb Sal I fragment was melted and the DNA therein ligated in substantial accordance with the teachings of Example 4. Plasmid pKC721 results from the ligation of the ~5.5 kb fragment. See Figure 8.

Example 7

Transformation of Streptomyces Griseofuscus and DNA Isolation Therefrom

A. Preparation of Protoplasts

The method set forth below was used in all experiments wherein transformation of Streptomyces griseofuscus was required.

A lyophil of Streptomyces griseofuscus C581 is obtained from the American Type culture Collection (ATCC), Rockville, MD 20852 under the accession number ATCC 23916. The lyophilized culture is used to inoculate 10 ml of TSB broth in a 50 ml flask. The culture is incubated at 29°C in a gyratory incubator overnight.

One half ml of a fully grown overnight culture of Streptomyces, homogenized, was used to inoculate 9.5 ml of TSB plus 0.5% glycine. The culture was incubated at 30°C for 24 hours. After homogenization with a tissue grinder, 0.5 ml of homogenate was used to inoculate 9.5 ml of fresh TSB supplemented with 0.5% glycine. The culture was incubated at 30°C for 24 hours. The culture was homogenized and transferred to a 50 ml sterile polystyrene centrifuge tube. The cells were pelleted by centrifugation, washed with 10 ml of P medium and resuspended in 10 ml of P medium with 1 mg/ml lysozyme, then incubated at room temperature for 15-30 minutes. Protoplast formation was monitored by examining small samples under a phase-contrast microscope. Protoplasts are spherical.

B. Protoplast Transformation

The protoplasts were centrifuged as before and washed once in P medium. The cells were resuspended in 10 ml of P medium and 150 $\mu$l of protoplasts for each transformation were placed in a 1.5 ml Eppendorf tube. Up to 10 µl of plasmid DNA in TE buffer were added with gentle mixing. One hundred µl of sterile 50% polyethylene glycol 1000 in P medium were added immediately, mixed well and allowed to sit at room temperature 30 seconds.

The transformation mixture was then plated on R2YE agar using an overlay of R2 soft agar. Plates were incubated 16 hours at 30°C. A second R2 soft agar overlay was applied containing a 25 µg/ml final concentration of apramycin. The plates were incubated at 30°C and transformants appeared 2-3 days later.

Individual colonies of transformants were cultured in 10 ml TSB containing apramycin (25 µg/ml) 2-3 days at 30°C.

C. Preparation of Total DNA

The following procedure was used to prepare total DNA from Streptomyces and thus supports several other examples. The culture was homogenized and aliquoted into 1.5 ml Eppendorf tubes. The tubes were centrifuged and the supernatants were removed. The pellets were resuspended in ~0.5 ml of medium containing 0.3M sucrose, 25 mM Tris (pH = 8.0), 25 mM EDTA, 5 mg/ml lysozyme, and 50 µg/ml RNase A. Following a 30 minute incubation at 37°C, 250 µl of 2% sodium dodecyl sulfate (SDS) was added and the mixture was vortexed for ~1 minute. 250 µl of neutral phenol:chloroform (phenol:chloroform 1:1 equilibrated vs. 0.1M Tris-HCl, pH 8.0) was added. (Equilibration refers to adding 0.1M Tris-HCl pH 8.0 until a biphasic solution forms.) The tube and its contents were vortexed for ~30 seconds. The aqueous phase was aspirated using a Pasteur pipette. The phenol/chloroform extraction was repeated four times at which point the interface was clear. A 0.1 volume of 3M sodium acetate was added. 1 volume of isopropanol was then added, and the contents were mixed and held at room temperature for 5 minutes. The tubes were then centrifuged in an Eppendorf table-top centrifuge for 5 minutes. After centrifugation, the upper phase was aspirated. After an additional 1 minute centrifugation the remaining liquid was aspirated, leaving only the pellet. The pellet was dissolved in 500 µl of TE. 25 µl of 0.1M spermine was added and the tubes were mixed at room temperature for 5 minutes. Centrifugation at room temperature for 10 minutes resulted in precipitation of the DNA. After aspiration of the supernatant the pellet was resuspended in 300 µl final volume comprising 1 mM MgCl₂ and 0.3M sodium acetate. 700 µl of ethanol were added, the contents were mixed then centrifuged of room temperature for 5 minutes. The supernatant was decanted, after which the DNA pellet was washed with ethanol and dried under vacuum. The DNA pellet was then resuspended in 50 µl TE.

D. Preparation of Streptomyces Plasmids

The method of Streptomyces plasmid preparation set forth below was used to prepare plasmid DNA from all Streptomyces transformants utilized in the present examples.

Approximately 5 ml of an overnight broth homogenate was centrifugally harvested. The cell pellet was resuspended to a final volume of 2 ml in STE containing approximately 0.3 mg lysozyme. The suspension was mixed and then incubated at 37°C for 1 hour. Four aliquots of 500 µl each were placed in separate 1.5 ml Eppendorf tubes.

250 µl of 0.3M NaOH containing 2% SDS was added to each tube. The tubes were vigorously vortexed, then heated at 70°C for 15 minutes. The tubes were allowed to cool to room temperature. Eighty µl of 1:1 phenol:chloroform were added. The tubes were vigorously vortexed, then centrifuged for 5 minutes. The aqueous phase containing the plasmid DNA was then harvested from each tube and placed in a fresh 1.5 ml Eppendorf tube. 70 µl of 3M sodium acetate and 700 µl of isopropanol were added to each tube. The tubes were mixed well, then centrifuged for 10 minutes. The supernatant was aspirated leaving only the pelleted DNA. The tube was centrifuged 1 minute longer and aspirated again to remove all isopropanol. Plasmid DNA was resuspended in 500 µl TE and 25 µl 0.1M spermine. The contents were mixed, then centrifuged for 10 minutes. The supernatant was discarded. The pellet was then washed in Keiser wash solution. After a 10 minute centrifugation, the supernatant was discarded and the pellet was washed with ethanol then dried under vacuum. The contents of all four tubes were pooled and resuspended in a total volume of 10 µl TE.

Example 8

Transformation of Streptomyces griseofuscus with Plasmid pKC721

Plasmid pKC721 was prepared as described in Example 6. Transformation procedures were carried out in substantial accordance with the teachings of Example 7. Transformants were selected on R2YE

containing apramycin (25 μg/ml).

Example 9

Construction of Plasmid pKC 761

Plasmid pKC761 was prepared by deleting the ~1.5 kb Fsp I fragment from plasmid pKC721. A restriction site and function map of plasmid pKC761 is provided in Figure 9.

Plasmid pKC721 was prepared by transforming E. coli JM109 with plasmid pKC721 DNA (Example 6). The transformation procedure was performed in substantial accordance with the method of Example 3 except that E. coli JM109 were used instead of E. coli DH5∝ and plasmid pKC721 was used instead of the plasmid pKC684/ pKC685 ligation mixture. Transformants were selected on TY agar containing apramycin (100 μg/ml).

Plasmid pKC721 was purified on a cesium chloride gradient in substantial accordance with the teachings of Example 2. 2 μg of cesium chloride purified plasmid pKC721 were digested with 12 U Fsp I in a total volume of 20 μl. Fsp I digestion buffer consists of 50 mM NaCl, 10 mM Tris-HCl (pH = 7.4), 10 mM MgCl₂, 10 mM DTT, and 100 μg/ml bovine serum albumin (Fsp I digestion buffer).

The Fsp I digest was separated using agarose gel electrophoresis (NuSieve GTG) and the ~4 Kb fragment was harvested from the gel as an approximately 40 μl gel plug. The gel was melted and the ~4 kb DNA was ligated in substantial accordance with the method taught in Example 4. Plasmid pKC761 resulted from the ligation of the ~4 kb DNA fragment.

Streptomyces griseofuscus C581 was transformed with plasmid pKC761 in substantial accordance with the protoplasting and transformation methods taught in Example 7. Transformants were selected on R2YE containing apramycin (25 μg/ml) as taught in Example 7. Isolated colonies were cultured and plasmids were isolated therefrom in substantial accordance with the teachings of Example 7.

Plasmid pKC761 DNA was then transformed into E. coli (JM109) and transformants were selected on TY containing apramycin (100 μg/ml). The transformation of E. coli was described in Example 4. Plasmid pKC761 was isolated in substantial accordance with the teaching of Example 2. Restriction endonuclease mapping to confirm the structure of plasmid pKC761 can be performed guided by the restriction map provided in Figure 9.

Example 10

Construction of Plasmid pKC978

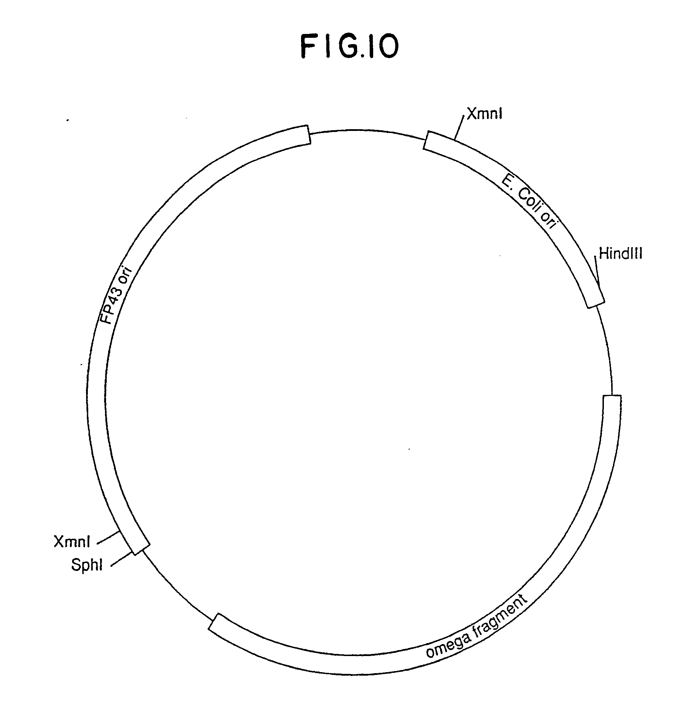

A restriction site and function map of pKC 978 is provided in Figure 10.

(A) Source of Plasmid pKC Omega Components

The omega fragment conferring spectinomycin resistance was purchased from Amersham Inc., 2636 S. Clearbrook Drive, Arlington Heights, IL 60005 (Amersham). The resistance gene is in cassette form, i.e., flanked by multiple restriction sites. The omega fragment was amplified by cloning it into pUC19 (Bethesda Research Laboratories, Inc., P.O. Box 577, Gaithersburg, MD 20760 (B.R.L.), catalog number 5364A) as described below.

(B) Amplification of the Omega Fragment

1 μl of pUC19 was digested with 10 U of Sma I in a 20 μl reaction volume comprising 20 mM KCl, 6 mM Tris-HCl (pH 8.0), 6 mM MgCl₂, 6 mM DTT, 100 μg/ml bovine serum albumin, for 1 hour at 25° C. The Sma I digested pUC19 termini were then dephosphorylated to prevent self-ligation. Dephosphorylation was effected by addition of 480 μl H₂O and 0.15 U bacterial alkaline phosphatase (International Biotechnologies, Inc., P. O. Box 1565, New Haven, CT 06506) (IBI) and incubating the reaction at 70° C for 1 hour.

One μl of the omega fragment was ligated to 10 μl of the Sma I digested, alkaline phosphatase treated plasmid pUC19 by combining them in a 20 μl total volume also containing 2 μl of 10X ligase buffer, 2 μl 10 mM ATP, 2 μl H₂O, and 2 μl T4 DNA ligase (New England Biolabs). 5 μl of the ligation mixture and was used to transform Stratagene XL-1 Blue cells (Stratagene Corp., 3770 Tansey Street, San Diego, CA. 92121) in substantial accordance with the transformation procedure taught in Example 3(B). Example 3(B) was modified by substitution of the Stratagene XL-1 Blue cells for the E. coli pH5∝ cells and the ligation mixtures were different. Transformants were selected on TY spectinomycin (100 μg/ml).

Individual isolates were cultured in TY broth selecting for spectinomycin (100 μg/ml) resistance. Plasmids were isolated in substantial accordance with Example 3(C) except cells were cultured in TY containing spectinomycin at 100 μl/ml. Plasmid constructions were confirmed by restriction endonuclease mapping. A double-digest with EcoRI and Hind III confirmed the presence of the omega fragment in the plasmid designated pKC omega. Double digestion of plasmid pKC omega with EcoRI and Hind III resulted in an ~2.6 kb fragment and an ~2.0 kb fragment. A transformant which contained the omega insert was then inoculated into TY broth containing spectinomycin at 100 μg/ml for amplification. Stratagene XL-1 Blue pKC omega were harvested from the broth and the pKC omega plasmids were purified on a cesium chloride gradient in substantial accord with the method of Example 2.

(C) Isolation of the Spectinomycin Resistance Gene (SpcR) from Plasmid pKC Omega

Approximately 2 μg of plasmid pKC omega was digested with 10 U Hind III and 10 U Sph I in a total digestion volume of 20 μl. The digestion buffer comprised 100 mM NaCl, 10 mM Tris-HCl (pH 7.4), 10 mM MgCl₂, 10 mM DTT, and 100 μg/ml bovine serum albumin. Digestion occurred at 37°C for 1 hour. The ~1.8 kb Hind III/Sph I fragment of plasmid pKC omega was isolated using gel electrophoresis. A 1% agarose gel and TAE electrophoresis buffer were used. The ~1.8 kb Hind III/Sph I fragment was harvested from the gel, extracted with phenol, extracted with Sevag, ethanol precipitated, and resuspended in 10 μl TAE.

(D) Isolation of the FP43 Origin of Replication

Approximately 2 μg of plasmid pKC721 (Example 6) was digested with 10 U of Fsp I in a total volume of 20 μl. The digestion buffer comprised 50 mM NaCl, 10 mM Tris-HCl (pH 7.4), 10 mM MgCl₂, 10 mM DTT and 100 μg/ml bovine serum albumin. Digestion occurred at 37°C for 1 hour. After the 1 hour digestion of plasmid pKC721 with Fsp I, 1 μl of 2 mM NaCl and 10 U Sph I were added, then the tube was incubated at 37°C for one hour. The entire digestion reaction was purified on a 1% NuSieve agarose gel using TAE as the running buffer. The phage FP43 origin of replication was harvested from the gel as the ~1.3 kb fragment.

(E) Isolation of E. Coli Replicon

Plasmid pACYC184 was obtained from the ATCC under accession number 37033. Plasmid pACYC184 carries the origin of replication from plasmid pI5A which enables it to co-exist with vectors like pBR322 and pUC19 that carry the Col E 1 origin. E. coli K-12 Pm191/pACYC184 ATCC accession number 37033 is chloramphenicol and tetracycline resistant. E. coli K-12 Pm191/pACYC184 was cultured in TY broth containing tetracycline (20 μg/ml) and chloramphenicol (20 μg/ml). Cells were harvested and plasmid preparations were performed in substantial accordance with the teachings of Example 2.

Approximately 2 μg of plasmid pACYC184 were digested with 10 U Pvu II and 10 U Hind III in a total volume of 20 μl. The digestion buffer consisted of 100 mM NaCl, 50 mM Tris-HCl (pH 8.0), 10 mM MgCl₂, and 100 μg/ml bovine serum albumin. Digestion was completed in 1 hour at 37°C. The digestion fragments were separated on a 1% NuSieve agarose gel using TAE as the running buffer. The E. coli replicon was recovered from the gel as the ~1 kb fragment. The ~1 kb Pvu II/Hind III fragment of plasmid pACYC184 was purified in substantial accordance with the teachings of Example 12(B).

(F) Assembly of Plasmid pKC978

3 μl of the purified omega fragment (step C), 3 μl of the phage FP43 origin of replication (step D), and 3 μl of the E. coli replicon (step E) were ligated in substantial accordance with the teachings of Example 3(A).

(G) Transformation of Streptomyces Griseofuscus With Plasmid pKC978

5 μl of the ligation mixture of step F were used to transform S. griseofuscus in substantial accordance with the teachings of Example 7. Transformants were selected on R2YE containing 100 μg/ml spectinomycin.

Plasmid pKC978 construction was confirmed as follows. Spectinomycin resistant S. griseofuscus colonies were cultivated in TSB containing 100 μg/ml spectinomycin for 4 days. Plasmids were prepared as taught in Example 7. Digestion of plasmid pKC978 with Hind III resulted in a single ~4.1 kb band. Digestion of plasmid pKC978 with Xmn I resulted in an ~2.9 kb fragment and an ~1.1 kb fragment. Protocols for Xmn I and EcoRI digestions were previously provided.

(H) Large Scale Preparation of Plasmid pKC978

Plasmid pKC978 was used to transform Stratagene XL-1 Blue E. coli in substantial accordance with the teachings of Example 3. Transformants were selected on TY containing 100 μg/ml spectinomycin. Plasmids were harvested from spectinomycin resistant clones as taught in Example 3(C). Restriction endonuclease mapping as described in step G confirmed the identity of plasmid pKC978. A clone confirmed to contain plasmid pKC978 was then utilized to prepare a large quantity of plasmid pKC978 as follows.

A 0.2 ml sample of a clone containing plasmid pKC978 was used to inoculate 1 1 of TY broth containing 100 μg/ml spectinomycin. The culture was incubated at 37°C for 15 hours. Plasmid pKC978 was harvested and purified on a cesium chloride gradient in substantial accordance with the teachings of Example 2.

Example 11

Southern Blot Analysis of S. griseofuscus Transformants

A. Preparation of the Cultures

Ten ml cultures of S. griseofuscus C581 and S. griseofusius transformants being analyzed for integrated versus antonomous plasmid presence were prepared by inoculating 10 ml of TSB with 0.1 ml of corresponding cultures prepared as previously set forth. The cultures were incubated overnight at 30°C in a gyratory water bath.

B. Restriction Endonuclease Digestion and Gel Electrophoresis

10 μl of the total DNA prepared as taught in Example 7 was digested with 12 U of Sma I for 1 hour at 25°C in a 20 μl final volume comprising 20mM KCl, 6mM Tris-HCl (pH 8.0), 6mM MgCl₂, 6mM 2-mercaptoethanol, and 100 μg/ml bovine serum albumin.

Plasmids were purified on a cesium chloride gradient in substantial accordance with the teaching of Example 2. Approximately 0.4 μg of each plasmid was digested with 12 U of Sma I using the same volume, digestion buffer, and digestion conditions as described above for total DNA.

Multiple gels of each sample DNA were run to allow hybridization with the panel of probes.

The Sma I digests prepared above were then electrophoresed on 0.7% agarose (NuSieve GTG) gels. Total DNA was applied as 15 μl of the total DNA Sma I digest. Plasmid DNAs were loaded as 1 μl of the Sma I digest. TAE was used as the gel electrophoresis buffer. 50 V were applied and the gels were run overnight. Molecular weights of restriction fragments were determined by comparison to migration distances of standards of known molecular weights.

C. Preparation of Probes

Probes were prepared as follows. Probes were prepared for plasmid pKC702 and plasmid pKC703. The following reagents were added to a 1.5 ml Eppendorf tube (sitting in ice): 1 μl (~0.5 μg) of the relevant plasmid DNA from which the probe was being produced, 5 μl of 10X nick-translation buffer [0.5M Tris-HCl (pH 7.2), 0.1M MgSO₄, 1mM DTT, and 50 U μg/ml bovine serum albumin]; 1 μl of 1mM dATP; 1 μl of 1mM dTTP; 1 μl of 1mM dGTP; 156 picomoles ∝ ³²PdCTP; 1 μg of test DNA; and water to achieve a final volume of 45 μl. The dideoxy nucleoside triphosphates (dATP, dTTP, dGTP, and ∝ ³²PdCTP) were purchased as aqueous solutions from the DuPont Company, NEN® Research Products, Customer Services, 549 Albany Street, Boston MA 02118. A 5 μl volume of a solution containing 5 U of E. coli DNA polymerase I and 5 U of DNase I was added. The nick translation mixture was gently mixed, then incubated on ice for 1 hour. 2 μl of 0.5M EDTA were added to terminate the reaction.

The specific activity of the plasmid DNA probe was determined after separating the nick translated DNA probes from unincorporated nucleoside triphosphates by trichloroacetic acid (TCA) precipitation.

D. Transfer of DNA Onto S & S Nytran Membranes

(1) DNA fractionation

18

DNA was fractionated on an 0.7% agarose gel. A Tris-acetate (TAE) buffer consisting of 40 mM Tris-acetate 1 mM EDTA, ph 8.0, was used as the electrophoresis buffer. DNA fragments were stained after electrophoresis with 0.5 $\mu$g/ml ethidium bromide.

(2) Membrane preparation for DNA transfer

S & S NYTRAN nylon membranes were purchased from Schleicher and Schuell, Inc, Keene, NH 03431 and used as the preferred hybridization membranes. The membranes were prepared by floating them in deionized $H_2O$, then submerging them to wet thoroughly. The membranes were rinsed and soaked in water until needed.

(3) DNA fragmentation

DNA fragmentation was accomplished by immersion of the gels in 0.25N HCl for 8 minutes at room temperature.

(4) Gel equilibration in transfer buffer

The gel was rinsed in deionized water, then soaked in two changes of 0.4M NaOH for 30 minutes. A 2 fold volume of 0.4M NaOH per volume of gel is preferred.

(5) DNA transfer to NYTRAN membranes by capillary action

DNA was transferred from the 0.7% agarose gel to the NYTRAN membrane by capillary action. 0.4M NaOH was used as the transfer buffer. 3 pieces of Whatman blotting paper were cut to generate blotting paper 6" larger than the gel. The blotting paper was saturated with transfer buffer and placed on a glass plate. The blotting paper-glass plate was placed in the bottom of a glass baking dish and the 0.7% agarose gel was then placed on the blotting paper. The NYTRAN membrane was placed atop the gel and 3 more pieces of blotting paper (precut to fit the gel) were placed atop the NYTRAN membrane. Parafilm® strips were placed along the gel to prevent contact between the upper and lower layers of blotting paper. A one inch stack of paper towels was placed atop the upper layer of blotting paper. A volume of transfer buffer sufficient to saturate the lower layer of blotting paper was added to the baking dish. The transfer procedure was completed by overnight incubation at room temperature. Paper towels were replaced as they became saturated.

(6) Membrane preparation for hybridization

After the transfer process was completed, the membrane was blotted, washed twice in SSPE (0.18M NaCl, 10 mM phosphate buffer (pH 7.7), 1 mM EDTA) at room temperature for 5 minutes. The membrane was then wiped with a latex-gloved finger to remove any agarose retained on the membrane from its contacting the gel during the capillary transfer.

(7) Hybridization protocol
(A) Prehybridization - The membrane (from step 6) was placed in a heat-sealable polyethylene bag and approximately 0.25 ml/cm² prehybridization buffer was added. Prehybridization buffer consists of 5X SSPE, 5X Denhardt's solution, 1% SDS, 20 $\mu$g/ml salmon testes DNA (fragmented, denatured, and phenol extracted prior to use), and 10 $\mu$g/ml Poly-A RNA. Denhardt's solution consists of 0.02% ficoll, 0.02% polyvinylpyrrolidone, and 0.02% bovine serum albumin. Salmon testes DNA was purchased from Sigma Chemical Co., St. Louis, MO 63178. The membrane was incubated in prehybridization buffer for 2 hours at 42° C.
(B) The pre-hybridization solution was removed from the container. Hybridization buffer was then added. Hybridization buffer consists of 5X SSPE, 1% SDS, 50% formamide (pH 7.4), 20 ug/ml fragmented, denatured DNA, and 10% dextran sulfate 500.
(C) Hybridization - Probes were prepared as described above (Example 11C). Probes were denatured by boiling in TE buffer for 5 minutes, then immediately placing them on ice. A volume of each probe, pre-calculated to contain $10^6$ cpm/probe, was then added to each membrane being probed.

Example 12

## Construction of Plasmid pKC1062

### A. Preparation of Plasmid pKC761 Fragment

Plasmid pKC761 was prepared as described in Example 9. Approximately 3 $\mu$g of plasmid pKC761 was digested with 10 U of Ase I (NEB) in a 20 $\mu$l digestion volume comprising 150 mM KCl, 10 mM Tris (pH 7.5), 10 mM MgCl$_2$, and 100 $\mu$g/ml bovine serum albumin for 1 hour at 37°C. The digest was phenol extracted, Sevag extracted, and ethanol precipitated. The resultant DNA precipitate was resuspended in 10 $\mu$l of TE. The DNA was then digested with 10 U Sph I in a 20 $\mu$l digestion volume comprising Sph I digestion buffer. The digest was separated using agarose (1% NuSieve) gel electrophoresis. The ~3.64 kb fragment was harvested from the gel, extracted, precipitated with ethanol and resuspended in 20 $\mu$l TE.

### B. Preparation of Lac∝ and MCS from Plasmid pUC19

The lac∝ and multiple cloning site (MCS) of plasmid pUC19, (BRL catalog #5364A) were prepared as follows. ~3 $\mu$l of pUC19 were digested with 10 U of Hae II in a total digestion volume of 20 $\mu$l. Hae II digestion buffer consists of 100 mM NaCl, 50 mM Tris-HCl (pH 8.0), 10 mM MgCl$_2$, and 100 $\mu$g/ml bovine serum albumin. The Hae II digested plasmid pUC19 was separated on an ~1% agarose gel (NuSieve). The ~0.445 fragment comprising the lac∝ and MCS was harvested from the gel, extracted, precipitated, and resuspended in ~20 $\mu$l TE.

### C. Synthesis of Linkers

Linkers 1842 and 1835 were prepared using conventional oligonucleotide synthetic methodology which is well known in the art of molecular biology. See Current Protocols in Molecular Biology, Vol. 1, Unit 2.11, 2.12, (ed. Ausubel, F., et al., Wiley Interscience Publisher, 1989). Linker 1842 consists of the product formed when TATAATATTATGCATGGCGC and CATGCATAATATTA were annealed. Linker 1835 consists of the product formed when CGCTAGCAATATTCATG and AATATTGCTAGCGGCGC were annealed.

The method of annealing single stranded oligonucleotide sequences is well known in the art. Briefly, the single stranded sequences being annealed were mixed at ~500 nanograms each, boiled at 55°C for 5 minutes and allowed to return to room temperature. Ligase buffer (Example 4) was used as the solvent for the annealing process and the final volume of the annealing process was ~20 $\mu$l.

### D. Construction of Plasmid pKC1062

1 $\mu$l of the ~0.445 kb pUC19/Hae II fragment, 1 $\mu$l of the ~3.64 kb pKC761/Ase I, Sph I fragment, 5 $\mu$l of linker 1842 (annealed) and 5 $\mu$l of linker 1835 (annealed) were ligated in substantial accordance with the teaching of Example 4. The final ligation volume was 20 $\mu$l.

### E. Transformation of E. Coli with Plasmid pKC1062

E. coli DH5∝ were transformed with 5 $\mu$l of the ligation reaction in substantial accordance with the teachings of Example 4B. Blue colonies were selected on TY containing apramycin (100 $\mu$g/ml), 5-bromo-4-chloro-3-indolyl-$\beta$-D-galactoside (X-gal) (0.008%), isopropylthiogalactoside (IPTG) (0.0002 mM), and MgSO$_4$ (0.01%). Transformants were grown in TY broth and subjected to restriction endonuclease mapping to confirm the structure plasmid pKC1062. Plasmids were isolated from transformants as described in Example 4B. Digestion of plasmid pKC1062 with Ssp I generates fragments of ~3.5 kb and ~0.5 kb. A restriction site and function map of plasmid pKC1062 is provided in Fig. 10.

## Claims

1. A DNA compound comprising the origin of replication isolated from phage FP43.

2. The DNA compound of claim 1 that is the ~1.3 kb Fsp I fragment.

3. A recombinant DNA vector comprising the DNA compound as claimed in Claim 1 or 2.

4. The recombinant DNA vector of Claim 3 that is pKC703.

5. The recombinant DNA vector of Claim 3 that is pKC721.

6. The recombinant DNA vector of Claim 3 that is pKC761.

7. The recombinant DNA vector of Claim 3 that is pKC978.

8. The recombinant DNA vector of Claim 3 that is pKC1062.

9. An organism transformed with a recombinant DNA vector as claimed in any one of Claims 3-8.

10. The organism of Claim 9 selected from the group consisting of Streptomyces, Chainia, Saccharopolyspora, and Streptoverticillium.

11. A process for preparing a recombinant DNA vector comprising the origin of replication of phage FP43, said process comprising inserting the ~1.3 kb Fsp I restriction fragment of phage FP43.

# FIG.1

# FIG.2

# FIG.3

# FIG.4

# FIG.5

# FIG. 6

# FIG.7

# FIG.8

# FIG.9

# FIG.IO

# FIG.II